(19) Europäisches Patentamt
European Patent Office
Office européen des brevets

(11) Veröffentlichungsnummer : **0 108 720**
**B1**

(12) # EUROPÄISCHE PATENTSCHRIFT

(45) Veröffentlichungstag der Patentschrift : **04.03.87**

(51) Int. Cl.⁴ : **C 07 D303/28**, C 07 C 43/188, C 08 G 59/04

(21) Anmeldenummer : **83810499.0**

(22) Anmeldetag : **31.10.83**

(54) **Neue cycloaliphatische Diallyläther.**

(30) Priorität : **04.11.82 CH 6414/82**

(43) Veröffentlichungstag der Anmeldung : **16.05.84 Patentblatt 84/20**

(45) Bekanntmachung des Hinweises auf die Patenterteilung : **04.03.87 Patentblatt 87/10**

(84) Benannte Vertragsstaaten : **CH DE FR GB LI**

(56) Entgegenhaltungen :
**CH-A- 476 787**
**CH-A- 625 796**
**DE-A- 1 643 295**
**DE-A- 2 947 734**
**US-A- 3 927 116**
**US-A- 4 342 673**
**CHEMICAL ABSTRACTS, Vol. 96, No. 13, 29. März 1982, Columbus, Ohio, USA BATOG, A.E., STEPKO, O.P., VARIVODA, A.A. "Glycidyl ethers of alcohols" Seite 691, Spalte 2, abstract-no. 104 067a**
**CHEMICAL ABSTRACTS, Vol. 96, No. 14, 5. April 1982, Columbus, Ohio, USA ORLOWSKI, JAN A. "1,4-Bis (3-methacryloyl-2-hydroxypropoxy) methyl)-cyclohexane" Seite 392, Spalte 1, abstract-no. 110 185d**
**Die Akte enthält technische Angaben, die nach dem Eingang der Anmeldung eingereicht wurden und die nicht in dieser Patentschrift enthalten sind.**

(73) Patentinhaber : **CIBA-GEIGY AG**
**Klybeckstrasse 141**
**CH-4002 Basel (CH)**

(72) Erfinder : **Monnier, Charles E., Dr.**
**Ch. du Verger 16**
**CH-1752 Villars-sur-Glane (CH)**

**EP 0 108 720 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft Diallyläther der 1,1'-Dicyclohexandiole, welche zur Herstellung der entsprechenden Diglycidyläther mittels Epoxidation geeignet sind.

Die Diglycidyläther der 1,1'-Dicyclohexandiole stellen insofern wertvolle Epoxidharze dar, als sie sich durch eine niedrige Viskosität auszeichnen und dadurch insbesondere als Giessharze oder reaktive Verdünner verwendet werden können. Von diesen Diglycidyläthern ist der Diglycidyläther des 4,4'-Dihydroxy-1,1'-dicyclohexyls aus CH-A-476 787 bekannt.

Es wurde nun gefunden, dass man durch Epoxidierung von Diallyläthern der 1,1'-Dicyclohexandiole, die neue Verbindungen darstellen, zu Epoxidharzen gelangt, die im Vergleich zu den aus den entsprechenden Diolen und mit Epichlorhydrin hergestellten Epoxidharzen halogenfrei sind.

Gegenstand der Erfindung sind somit unsubstituierte oder alkylsubstituierte Diallyläther der Formel I

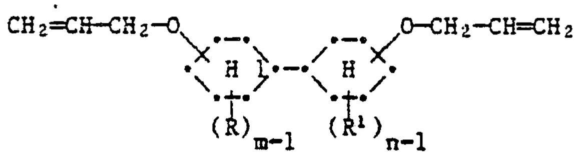

(I)

worin m und n unabhängig voneinander je für die Zahl 1, 2 oder 3 stehen, R und $R^1$ unabhängig voneinander je ein Alkyl mit 1-4 C-Atomen bedeuten, die beiden Allyläthergruppen unabhängig voneinander je in 1-, 2-, 3-, 4-, 5- oder 6-Stellung gebunden sind und wobei die Alkylgruppe und die Allyläthergruppe nicht am gleichen C-Atom gebunden sind.

Von den erfindungsgemässen Verbindungen stellen die unsubstituierten 2,2'-, 3,3'-, 4,4'-, 2,3'-, 3,4'- und 2,4'-Diallyläther der Formel I bevorzugte Verbindungen dar. Besonders interessant sind der 1,1'-Dicyclohexyl-4,4'-diallyläther und der -2,2'-diallyläther, insbesondere der 1,1'-Dicyclohexyl-4,4'-diallyläther.

Die erfindungsgemässen Verbindungen können hergestellt werden, indem man ein unsubstituiertes oder alkylsubstituiertes Diol der Formel II

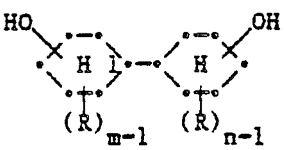

(II)

worin m, n, R und $R^1$ die gleiche Bedeutung wie in Formel I haben, mit Allylchlorid oder -bromid zum Diallyläther der Formel I umsetzt.

Die Diole der Formel II stellen teils bekannte und teils neue Verbindungen dar.

Die Diole des 1,1'-Dicyclohexans können beispielsweise nach dem in J. Am. Chem. Soc. *55,* 4234-7 (1933) beschriebenen Verfahren durch Hydrierung der entsprechenden Biphenole, beispielsweise 2,2'-, 3,3'- oder 4,4'-Biphenol, erhalten werden.

Die Umsetzung der Diole des 1,1'-Dicyclohexans mit Allylchlorid oder Allylbromid zum Diallyläther der Formel I kann beispielsweise vorteilhaft mittels der Phasentransferkatalyse im alkalischen Medium und in Gegenwart eines Phasenumwandlungskatalysators durchgeführt werden. Ein solches Verfahren ist beispielsweise in der EP-Anmeldung 046 731 beschrieben.

Die Diallyläther der Formel I stellen nicht nur wertvolle Ausgangsstoffe zur Herstellung von Epoxidverbindungen nach dem Epoxidationsverfahren dar, wobei im Vergleich zur Herstellung von Epoxidharzen mittels Epichlorhydrin halogenfreie Epoxidharze erhalten werden, sondern sie sind auch wertvolle Monomere, die allein oder in Mischung mit anderen polymerisierbaren Monomeren, wie beispielsweise Diallylphthalat, Triallylcyanurat oder Styrol, in vernetzte Polymeren überführt werden können.

Die Epoxidierung der C=C-Doppelbindungen in den Diallyläthern der Formel I kann nach einer der üblichen Methoden, wie Behandeln der Diallyläther mit epoxidierenden Mitteln, erfolgen, wie zum Beispiel mit Hilfe von organischen Persäuren, wie Peressigsäure, Perpropionsäure, Benzopersäure, oder auch $H_2O_2$ oder Hydroperoxide, wie tert.-Butylhydroperoxid. Ferner kann man auch Gemische aus $H_2O_2$ und organischen Säuren, wie Ameisensäure, oder vorzugsweise Nitrilen, wie Benzonitril oder Acetonitril, oder Säureanhydriden, wie Essigsäureanhydrid oder Bernsteinsäureanhydrid, verwenden.

Die folgenden Beispiele beschreiben die Erfindung näher. Wenn nichts anderes vermerkt ist, bedeuten Teile Gewichtsteile.

Beispiel 1

a) 1,1'-Dicyclohexyl-4,4'-diallyläther

In einem 1,5 Liter Sulfierkolben werden 97,0 g (0,5 Mol) 1,1'-Dicyclohexyl-4,4'-diol, 200 g (5 Mol) Natronlauge und 34,0 g (10 Mol %) Tetrabutylammoniumhydrogensulfat in 500 ml Toluol vorgelegt und auf 80 °C erwärmt. Innerhalb 1 1/2 Stunden werden 302,5 g (2,5 Mol) Allylbromid unter starkem Rühren bei dieser Temperatur zugetropft. Nach Beendigung des Zutropfens wird das Reaktionsgemisch noch während 2 1/2 Stunden gerührt ; anschliessend wird das Reaktionsgemisch mit 500 ml Wasser versetzt. Die wässerige Phase wird abgetrennt und noch 2 mal mit je 200 ml Toluol extrahiert. Danach werden die vereinigten Toluolphasen über $NaSO_4$ getrocknet, filtriert und eingeengt. Es resultieren 132,22 g (95 % der Theorie) 1,1'-Dicyclohexyl-4,4'-diallyläther als leicht gelbliche Flüssigkeit.

NMR-Spektrum ($CDCl_3$) : 0,8-2,1 ppm m 20H (Cyclohexyl-H) ;
3,8-4,0 m
4 H (—O—$CH_2$—) ; 4,8-5,3 m 4 H ($CH_2$=C—) ; 5,4-6,1 m 2 H (—C<u>H</u>=C)

IR-Spektrum (Film) : 3 080, 1 640, 1 450, 1 080, 1 020 $cm^{-1}$.

b) 1,1'-Dicyclohexyl-4,4'-diglycidyläther

In einem 750 ml Sulfierkolben werden 70,0 g (0,251 Mol) 1,1'-Dicyclohexyl-4,4'-diallyläther, 76,2 g (1,9 Mol) Acetonitril und 210 ml Methanol vorgelegt. Innerhalb 1 1/2 Stunden werden total 48,8 g (1,0 Mol) 70 %ige Wasserstoffperoxid in Portionen bei 40-45 °C hinzugetropft, wobei der pH-Wert konstant auf 9.0 gehalten wird. Nach Zugabe des Wasserstoffperoxids wird das Reaktionsgemisch während 4 1/2 Stunden weitergerührt und aufgearbeitet, d. h. mit $NaHSO_3$ von Peroxid befreit, das Methanol am Vakuum entfernt und der Rückstand in etwa 200 ml Chloroform aufgenommen und mit Wasser gewaschen, getrocknet und eingeengt. Es resultieren 64,75 g (83,12 % der Theorie) 1,1-Dicyclohexyl-4,4'-diglycidyläther als leicht gelbliches, niederviskoses Harz mit einem Epoxidgehalt von 5,43 Aequivalenten/kg (83,63 % der Theorie).

Viskosität (25 °C) : 125 mPa · s.

Beispiel 2

a) 1,1'-Dicyclohexyl-3,3'-diallyläther

In einem 200 ml Sulfierkolben mit Rührer, Tropftrichter, Kühler und Thermometer werden 10,00 g (0,050 Mol) 1,1'-Dicyclohexyl-3,3'-diol, 20,62 g (0,515 Mol) Natriumhydroxid, 3,51 g Tetrabutylammoniumhydrogensulfat und 50 ml Toluol vorgelegt und auf 70 °C erwärmt. Unter starkem Rühren werden 31,18 g (0,258 Mol) Allylbromid hinzugetropft. Nach 4 Stunden wird das Reaktionsgemisch in einen Scheidetrichter übergeführt und mehrmals mit Wasser gewaschen, dann getrocknet und eingeengt. Nach Destillation im Kugelrohr bei 120-125 °C/0,046 mbar werden 10,45 g (75,07 % der Theorie) 1,1'-Dicyclohexyl-3,3'-diallyläther als praktisch farbloses Oel erhalten.

IR-Spektrum (Film) : 3 080, 2 920, 2 860, 1 450, 1 350, 1 080, 920 $cm^{-1}$.

NMR-Spektrum ($CDCl_3$) : 0,9-2,5 m 20 H (Cyclohexyl-H) ; 4,0 m 4 H (—$OCH_2$—) ;
5,0-5,5 m 4 H (—C<u>H</u>$_2$—CH) ; 5,5-6,0 m 2 H (C<u>H</u>=C).

Elementaranalyse :
$C_{18}H_{30}O_2$(MG = 278,44)
berechnet : C = 77,65 % H = 10,86 %
gefunden : C = 77,80 % H = 10,3 %

b) 1,1'-Dicyclohexyl-3,3'-diglycidyläther

In einem 100 ml Sulfierkolben mit Rührer, Thermometer, Kühler und Tropftrichter und pH-Impulsomat werden 9,80 g (0,035 Mol) 1,1'-Dicyclohexyl-3,3'-diallyläther, 10,64 g (0,259 Mol) Acetonitril, 23,0 ml Methanol und 0,21 g Dinatriumhydrogenphosphat vorgelegt und auf 55 °C erwärmt. Innerhalb 90 Minuten werden bei dieser Temperatur langsam 6,79 g (0,14 Mol) 70-%ige Wasserstoffperoxidlösung hinzugetropft, wobei das pH auf 8,5-9,0 mit 20 %-iger Natronlauge eingestellt wurde. Nach beendeter Zugabe wird das Raktionsgemisch noch während 24 Stunden ausreagieren gelassen. Anschliessend werden 50 ml Wasser hinzugefügt und das Gemisch 4 mal mit 100 ml Chloroform extrahiert. Die vereinigten Chloroformphasen werden mit Natriumbisulfit und Wasser peroxidfrei gewaschen und mit Natriumsulfat getrocknet und eingeengt. Es resultieren 10,50 g (97,15 % der Theorie) 1,1'-Dicyclohexyl-3,3'-diglycidyläther als klares, gelbliches Oel mit einer Viskosität von 270 cP/25 °C und einem Epoxidgehalt von 5,68 Aequivalenten/kg.

IR-Spektrum (Film) : 2 920, 2 860, 1 730, 1 600, 1 450, 1 350, 1 250, 1 090, 910, 850 $cm^{-1}$.

NMR-Spektrum ($CDCl_3$) : 0,8-2,0 m 20 H (Cyclohexyl-H) ;
2,4-2,8 m 4 H (C<u>H</u>$_2$—CH),
2,8-3,2 m 2 H (C<u>H</u>—$CH_2$—) ;
3,3-4,1 m 4 H (—$OCH_2$—).

**Patentansprüche**

1. Unsubstituierter oder alkylsubstituierter Diallyläther der Formel I

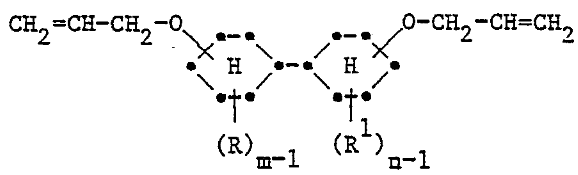

(I)

worin m und n unabhängig voneinander je für die Zahl 1, 2 oder 3 stehen, R und $R^1$ unabhängig voneinander je ein Alkyl mit 1-4 C-Atomen bedeuten, die beiden Allyläthergruppen unabhängig voneinander je in 1-, 2-, 3-, 4-, 5- oder 6-Stellung gebunden sind und wobei die Alkylgruppe und die Allyläthergruppe nicht am gleichen C-Atom gebunden sind.

2. Unsubstituierter 2,2'-, 3,3'-, 4,4'-, 2,3'-, 3,4'- und 2,4'-Dialyläther der Formel I gemäss Anspruch 1.

3. 1,1'-Dicyclohexyl-4,4'-diallyläther gemäss Anspruch 1.

4. 1,1'-Dicyclohexyl-3,3'-diallyläther gemäss Anspruch 1.

**Claims**

1. An unsubstituted or alkyl-substituted diallyl ether of formula I

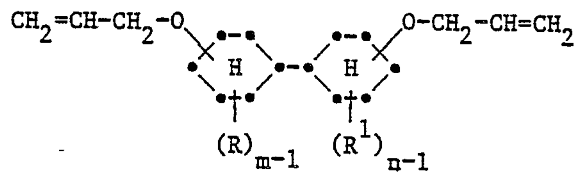

(I)

wherein each of m and n independently of the other is 1, 2 or 3, each of R and $R^1$ independently of the other is $C_1$-$C_4$alkyl, and each of the two allyl ether groups independently of the other is bound in the 1-, 2-, 3-, 4-, 5- or 6-position, the alkyl group and the allyl ether group not being bound to the same carbon atom.

2. An unsubstituted 2,2'-, 3,3'-, 4,4'-, 2,3'-, 3,4'- or 2,4'-diallyl ether of formula I according to claim 1.

3. 1,1'-Dicyclohexyl-4,4'-diallyl ether according to claim 1.

4. 1,1'-Dicyclohexyl-3,3'-diallyl ether according to claim 1.

**Revendications**

1. Ethers diallyliques non substitués ou porteurs d'un ou plusieurs radicaux alkyles, qui répondent à la formule I.

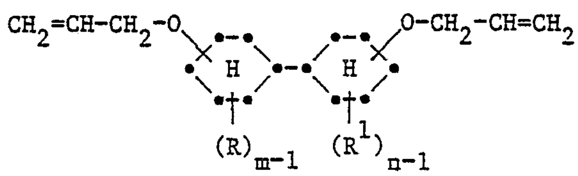

(I)

dans laquelle m et n représentent chacun, indépendamment l'un de l'autre, un nombre égal à 1, à 2 ou à 3, R et $R^1$ représentent chacun, indépendamment l'un de l'autre ou les uns des autres, un radical alkyle contenant de 1 à 4 atomes de carbone, les deux radicaux allyloxy se trouvent chacun, indépendamment l'un de l'autre, en l'une des positions 1, 2, 3, 4, 5, et 6, et le radical alkyle et le radical allyloxy ne se trouvent pas sur le même atome de carbone.

2. Ethers diallyliques de formule I selon la revendication 1 qui ne portent pas de substituants et dont les deux radicaux allyloxy se trouvent aux positions 2,2', 3,3', 4,4', 2,3', 3,4' et 2,4'.

3. Ether diallylique selon la revendication 1, en l'espèce le diallyloxy-4,4' bicyclohexyle-1,1'.

4. Ether diallylique selon la revendication 1, en l'espèce le diallyloxy-3,3' bicyclohexyle-1,1'.